# EUROPEAN PATENT APPLICATION

(11) **EP 2 620 885 A2**
(43) Date of publication of application: **31.07.2013**
(21) Application number: 13151430.9
(22) Date of filing: 16.01.2013
(51) Int. Cl.: G06F 19/00

(54) **Medical image processing apparatus**

(30) Priority: 30.01.2012 US 201213361003; 09.10.2012 JP 2012224021
(71) Applicant: Kabushiki Kaisha Toshiba, Minato-ku, Tokyo 105-8001 (JP); Toshiba Medical Systems Corporation, Otawara-shi, Tochigi 324-8550 (JP)
(72) Inventor: Bird, Ian Douglas, Edinburgh, EH6 5NP (GB); Roberts, Colin John, Edinburgh, EH6 5NP (GB)
(74) Representative: Lewis, Darren John

(57) **Abstract**

According to one embodiment, a medical image processing methods includes setting a portion of interest in medical image data in accordance with an instruction from a user, selecting at least one candidate preset corresponding to at least one candidate observation target according to the portion of interest from a plurality of presets using a pixel value of a pixel included in the set portion of interest, each of the plurality of presets defining a correspondence relationship between an observation target and a display condition, and displaying the at least one selected candidate preset.

## Description

### FIELD

Embodiments described herein relate generally to a medical image processing apparatus.

### BACKGROUND

In the medical field, medical image data such as 2D image data and 3D image data are acquired by various image diagnostic apparatuses. These image diagnostic apparatuses are called modalities in the medical field. As the image diagnostic apparatuses, an X-ray diagnostic apparatus, an X-ray computed tomography apparatus (CT scanner), a magnetic resonance imaging apparatus, an ultrasonic diagnostic apparatus, a positron emission tomography apparatus (PET scanner), and the like are known. The 2D image data include not only general X-ray images but also 2D images generated from 3D image data (that is, volume data). Examples of the 2D images are a "slice" obtained by CT scan and a "slab" obtained by MPR (Multi-Planar Reconstruction) of volume data from CT scan. Time-series 3D study is called 4D study, as is well known. The 4D study has time as the fourth dimension. For example, the time-series perfusion of an abdominal organ is measured using 4D DCE-CT (Dynamic Contrast Enhanced CT). There is also known an image diagnostic apparatus formed by combining different modalities, like a PET/CT apparatus that combines a PET scanner and a CT scanner. Merging a plurality of types of medical images obtained for one patient by such an image diagnostic apparatus is known. The merged display is sometimes called 5D study.

A medical image display application displays an image based on a medical image by gray scale. The gray scale is used in monochrome display, and a color scale is used in color display. For the sake of descriptive convenience, the medical image display application is assumed to use monochrome display. The density value of an image displayed by the gray scale can be restored to the pixel value (for example, Hounsfield Unit (HU)) of the original medical image generated by the image diagnostic apparatus. A human being poorly identifies shades. A human being can identify only about 20 to 30 density differences (contrasts). Hence, instead of displaying the pixels within the full pixel value range by gray scale at once, only pixels in the pixel value range to which the observation target belongs are displayed by gray scale. The display conditions usually include "window width" and "window level". The window level is the center value of the pixel value range of the observation target. The window width is defined by the pixel value range of the observation target. In the monochrome display, a pixel having a pixel value smaller than the window width is displayed in black, and a pixel having a larger pixel value is displayed in white. For example, a bone has a dense structure. Pixels corresponding to a bone in CT image data have high Hounsfield unit (HU) values. To display bones in the CT image data at a high contrast, the window level is set to a high value, for example, about 350 HU. Bones have a wide HU value range of, for example, 1,200 HU, as is well known. Hence, the window width is relatively set wide.

A specific type of tissue or tissue character will be referred to as an observation target. A combination of an observation target and display conditions suitable for the observation target is called a "preset" in the field of medical image processing. Note that the display conditions are parameters associated with image display and include the window level and the window width. Various presets are stored in every modality. In accordance with an observation target, a user can arbitrary select, from various presets, a preset suitable for displaying the observation target. The term "preset" is derived from the fact that the display conditions are initially determined and stored for user selection. In the present field of medical image processing, "preset" is used as a more general term indicating any set of display conditions that are not necessarily determined in advance. For example, several applications adjust or set the window level and the window width by on-the-fly during the period of observation by the user. The term "preset" is assumed to be used complying with the standard manner of the medical field and include both display conditions initially determined and those determined by on-the-fly.

A variety of clinical applied technologies have been developed for various modalities. For example, CT scan is used to inspect various kinds of tissues from a relatively hard tissue and a relatively soft tissue. In the CT, the voxel value of a voxel included in volume data has a very wide HU value range. As a result, many presets are found out, and each preset is used in a specific clinical study. In the CT or MR study, a contrast agent is sometimes used to enhance the clinical target. Various contrast media having various characteristics have been developed for the respective modalities. For example, there are presets optimized to angiographic images acquired using specific contrast agent. Display conditions are stored, which are optimized in advance in accordance with the characteristics of these clinical applications. To assist the user in selecting an appropriate preset, the display conditions are displayed in association with the name of a clinically associated observation target.

Examples of presets for 2D X-ray images, MPR images based on CT volume data, and the like are the abdomen, abdominal blood vessels, blood vessels, bones (hard), bones (soft), calcium, fat, liver, liver (contrast imaging), lungs, mediastinum, pectoral blood vessels, and head.

Along with the evolution and specialization of clinical knowledge, still more presets are defined and accumulated. This makes it difficult for the user to select an optimum preset from the enormous number of presets. For example, the user selects a preset based on the subject, the type of medical image data, the presence/absence of medical image data calibration, the medical image data calibration method, the observation target, and the like.

### BRIEF DESCRIPTION OF THE DRAWINGS

FIG. 1 is a view showing the arrangement of a computer system according to the embodiment;
FIG. 2 is a view schematically showing the internal structure of a file complying with the DICOM standard according to the embodiment;
FIG. 3 is a perspective view showing the outer appearance of an X-ray computed tomography apparatus according to the embodiment;
FIG. 4 is a block diagram showing the arrangement of a computer according to the embodiment;
FIG. 5 is a view showing the detailed arrangement of the computer shown in FIG. 4;
FIG. 6 is a view showing a slab MPR image based on CT volume data to which a preset for the abdomen according to the embodiment is applied;
FIG. 7 is a view showing a slab MPR image based on the same CT volume data as in FIG. 6 to which a preset for the bones according to the embodiment is applied;
FIG. 8 is a view showing a slab MPR image based on the same CT volume data as in FIGS. 6 and 7 to which a preset for the lungs according to the embodiment is applied;
FIG. 9 is a flowchart showing the typical procedure of candidate preset display processing executed by a processing engine shown in FIG. 5;
FIG. 10A is a view showing the schematic outlines of a human body including several major organs on which a current display region designated by the user is illustrated so as to explain processing in step S5 of FIG. 9;
FIG. 10B is a view showing the schematic outlines of a human body including several major organs on which a display region zoomed in more than in FIG. 10A is illustrated so as to explain processing in step S5 of FIG. 9;
FIG. 11 is a view for explaining designation of a portion of interest performed in step S5 of FIG. 9;
FIG. 12A is a view showing a conventional list in which all presets are displayed;
FIG. 12B is a view showing a candidate preset list displayed by a display shown in FIG. 5 when the user clicks on an air region on a display image;
FIG. 12C is a view showing a candidate preset list displayed by the display shown in FIG. 5 when the user clicks on a bone region on the display image;
FIG. 12D is a view showing a candidate preset list displayed by the display shown in FIG. 5 when the user clicks on a soft tissue region on the display image;
FIG. 12E is a view showing an advanced candidate preset list displayed by the display shown in FIG. 5 when the user clicks on a bone region on the display image;
FIG. 12F is a view showing an advanced candidate preset list displayed by the display shown in FIG. 5 when the user clicks on a soft tissue region on the display image;
FIG. 12G is a view showing a still advanced candidate preset list displayed by the display shown in FIG. 5 when the user clicks on a bone region on the display image;
FIG. 13A is a view showing a candidate preset list displayed by the display shown in FIG. 5 when the user clicks on an air region in a coronal slice image; and
FIG. 13B is a view showing a candidate preset list displayed by the display shown in FIG. 5 when the user clicks on a bone region in the coronal slice image.

### DETAILED DESCRIPTION

In general, according to one embodiment, a medical image processing apparatus includes a storage unit, a setting unit, a selection unit, and a display unit. The storage unit stores a plurality of presets. Each of the plurality of presets defines the correspondence relationship between an observation target and a display condition. The setting unit sets a portion of interest in medical image data in accordance with an instruction from a user. The selection unit selects at least one candidate preset corresponding to at least one candidate observation target according to the portion of interest from the plurality of presets using the pixel value of a pixel included in the set portion of interest. The display unit displays the at least one selected candidate preset.

A medical image processing apparatus, an image diagnostic apparatus, a computer system, a medical image processing program, and a medical image processing method according to this embodiment will now be described with reference to the accompanying drawing. To be specific, the medical image processing apparatus according to this embodiment is a standalone computer. As the computer according to this embodiment, a personal computer or a workstation is applicable. The medical image processing apparatus may be incorporated in the image diagnostic apparatus. The medical image processing program causes the medical image processing apparatus to execute functions required of several examples. At least one function according to this embodiment may be implemented by, for example, specialty hardware having the form of a special purpose integrated circuit such as an ASIC (Application Specific IC).

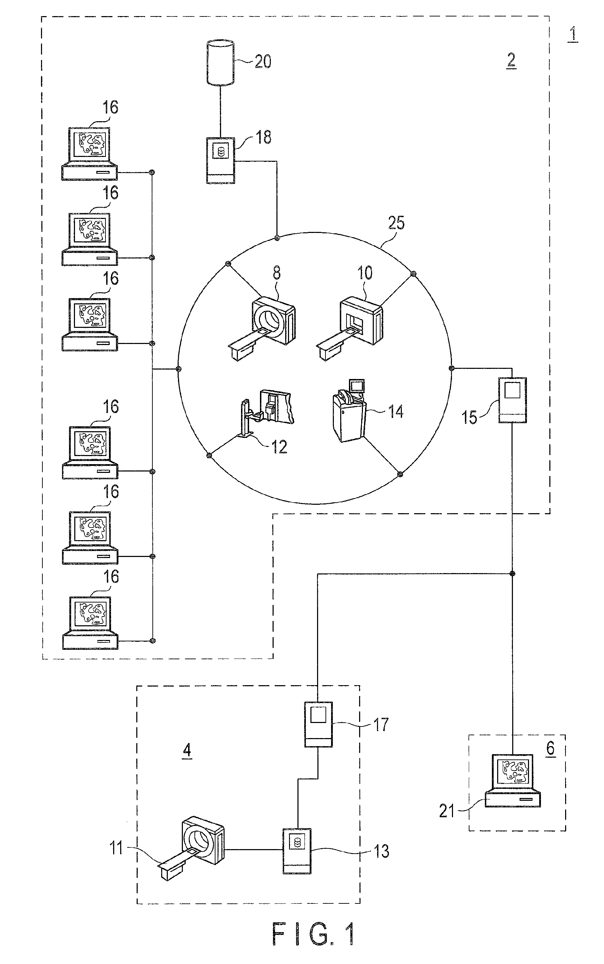

FIG. 1 is a view showing the network arrangement of a computer system 1 according to this embodiment. The computer system 1 includes a medical image processing apparatus, an image diagnostic apparatus, and associated devices. The computer system 1 has three networks, that is, a hospital network 2, a remote image diagnostic apparatus network 4, and a remote single user network 6. The hospital network 2 includes, for example, a plurality of image diagnostic apparatuses, a plurality of workstations 16, a common format file server 18, a file archive 20, and an Internet gateway 15. The hospital network 2 includes, for example, an X-ray computed tomography apparatus 8, a magnetic resonance imaging apparatus 10, a digital radiography (DR) apparatus 12, and a computed radiography (CR) apparatus as the image diagnostic apparatuses. These apparatuses are connected to each other via a LAN (Local Area Network) 25.

The remote image diagnostic apparatus network 4 includes an image diagnostic apparatus 11, a common format file server 13, and an Internet gateway 17. As the image diagnostic apparatus 11, an X-ray computed tomography apparatus, a magnetic resonance imaging apparatus, a digital radiography (DR) apparatus, a computed radiography (CR) apparatus, or the like is applicable. Note that FIG. 1 illustrates an X-ray computed tomography apparatus as the image diagnostic apparatus 11. The X-ray computed tomography apparatus 11 and the common format file server 13 are connected to the Internet gateway 17 and then connected to the Internet gateway 15 in the hospital network 2 via the Internet.

The remote single user network 6 includes a workstation 21. The workstation 21 includes an Internet modem (not shown). The workstation 21 is connected to the Internet gateway 15 in the hospital network 2 via the Internet.

The computer system 1 is configured to transmit various kinds of data using a standardized common format. For example, the X-ray computed tomography apparatus 8 initially generates an original medical image data set, for example, a 3D medical image data set. The medical image data set is coded by a standard image data format and transmitted to the file server 18 via the LAN 25. The file server 18 stores the medical image data set in the file archive 20. A user such as a radiographic technician, a consulting physician, or a research worker works at one of the plurality of workstations 16. The user requests, via the workstation 16, a desired medical image data set from the file server 18. The file server 18 reads out the requested medical image data set from the file archive 20 and offers the readout medical image data set to the workstation 16 of the request source via the LAN 25. In a similar manner, the computer 11 in the remote image diagnostic apparatus network 4 or the computer 21 in the remote single user network 6 can request a medical image data set stored in the file archive 20 or another apparatus in the network 2 and receive the requested medical image data set.

Software executed on the workstation 16 or 21 or via the workstation 16 or 21 is constituted by a common image data format. The standard image data format allows the computers 16 and 21, the file servers 13 and 18, the file archive 20, and the image diagnostic apparatuses 8, 10, 11, 12, and 14 to share the medical image data sets.

Currently, the most general image data format in medical image display applications is the "Digital Imaging and Communications in Medicine" format which is usually called DICOM. The DICOM standard is defined by the National Electric Manufactures' Association of America.

Medical image processing according to this embodiment is used in the computer system 1. In this case, the medical image processing according to this embodiment can be integrated with a standalone software application or a PACS (Picture Archiving and Communication System). The PACS is a computerized network in a hospital. The PACS stores a plurality of medical image data sets having different digital formats optimized in a single center archive. For example, the plurality of medical image data sets are stored in the DICOM format. Each medical image data set is associated with patient information such as a name and a date of birth stored in the file archive 20 or the like. The file archive 20 is connected to the network so that every user in the hospital can access the medical image data sets as needed. A user outside the hospital can be permitted to access the file archive 20 via the Internet.

A user can access the medical image data sets via a workstation. The workstation or the like displays still images based on a 2D medical image data set or a 3D medical image data set or a moving image based on a plurality of 3D medical image data sets.

In this embodiment, the medical image data set includes medical image data and additional data (metadata) concerning the medical image data. The medical image data is associated with the additional data by the image diagnostic apparatus or the like. Each medical image data set is handled as a DICOM file.

The DICOM file will be described later in detail.

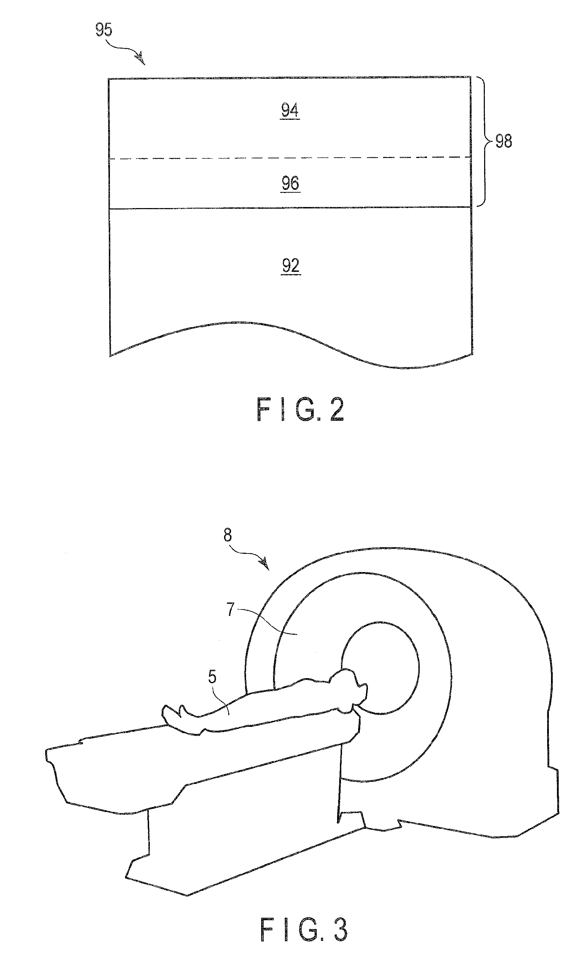

FIG. 2 is a view schematically showing the structure of a DICOM file 95. The DICOM file 95 includes a header portion 98 and an image data portion 92. The header portion 98 is divided into a first header portion 94 and a second header portion 96. The image data portion 92 stores medical image data having an image data format based on the DICOM standard. The header portion 98 stores additional data associated with the medical image data. The additional data is data about details that are generally used in the DICOM standard and can clearly be specified. Patient information, visit information, study information, result information, interpretation information, common composite image information, and modality specifying information are known as the details. The information included in the individual details is obligatory, conditional, or arbitrary. The particulars of the second header portion 96 are assigned to private tag information. In the private tag information, the user can arbitrarily determine the particulars. The private tag information is information that the user wants to associate with medical image data and that is not included in the first header portion 94 by the DICOM standard. The typical maximum size of the header portion 98 is 16 kbytes and limits the data amount stored in the header portion 98.

All apparatuses such as the image diagnostic apparatuses, the file servers, the file archive, and the computers included in the computer system 1 are designed to communicate in accordance with the DICOM standard.

The header portion 98 can store not only data about patient information but also additional data about information such as the presence/absence of use of a contrast agent, the modality, and the dose. The header portion 98 can also store additional data about a specific operation process of generating a 2D display image based on an original 3D medical image data set or 4D medical image data set. The header portion 98 can also store additional data such as the information of an instruction to a technician, an exemplary image, and a clinical report of doctor's findings. If additional data associated with medical image data cannot completely be stored in the header portion 98 of the DICOM file, several additional DICOM files may be used to store the additional data. The additional DICOM files are associated with the DICOM file including the medical image data. The additional data may be stored not only in the header portion of an additional DICOM file but also in the image data portion. To commonly execute write and read, a plurality of associated DICOM files may be generated in every study.

For example, the header portion stores additional data about at least one particular of modality information, contrast agent information, anatomical information to define a body portion of an imaging target, a diagnostic purpose, and a user's clinical status. More specifically, the particulars are
1. acquisition modality (image diagnostic apparatus)
2. the presence/absence of use of a contrast agent, and if used, the type of contrast agent
3. the state of a graphical user interface element to control the display conditions
4. 3D image data (volume data or multi-frame image data)
5. 2D image data
6. medical signal data acquired for a predetermined time
7. patient identification information (name, age, and other information to identify the patient)
8. case identification information/hospital (hospital, doctor, treatment type, and other information to identify the case)
9. directly input medical observation values or medical measurement values
10. medical history information of the patient
11. report information (texts, images, measurement values, and reference information linked to findings for specific positions of the patient)
12. other readable information (for example, an instruction or comment from one medical specialist to another medical specialist)
13. the identifiers of medical image data and other data objects included in the original medical image data set
14. a study number, series number, acquisition number, or any other management number to identify the original medical image data set
15. a file name, a URL, a network server identifier, or associated data to describe the location of the original medical image data set
16. volume rendering parameters such as transparency and color parameters
17. surface rendering parameters such as threshold parameters
18. tissue segmentation and selection information
19. the number of frames, the type of rendering, and the geometric and other characteristics of a moving image sequence

FIG. 3 is a perspective view schematically showing an example of the image diagnostic apparatus according to this embodiment. FIG. 3 illustrates the X-ray computed tomography apparatus (CT scanner) 8 as the image diagnostic apparatus. The X-ray computed tomography apparatus 8 acquires 3D scan data of the imaging region of a patient 5. The imaging region of the patient 5 includes at least one organ and other anatomical features. The imaging region of the patient 5 is placed in a circular opening 7 of the X-ray computed tomography apparatus 8. The X-ray computed tomography apparatus 8 acquires raw data about the imaging region of the patient 5. The raw data includes output data from 12 x 512 detection elements. The raw data is acquired for each view. The number of views set per rotation of the rotating frame is 1,000. The X-ray computed tomography apparatus 8 generates medical image data concerning the imaging region of the patient 5 based on the acquired raw data. The medical image data is typically defined by 3D coordinates. The 3D medical image data is known as volume data or multislice data. The X-ray computed tomography apparatus 8 generates a 2D display image based on the 3D medical image data. The generated display image is displayed typically on a display.

A modality such as an X-ray computed tomography apparatus, a magnetic resonance imaging apparatus, a PET apparatus, or an ultrasonic diagnostic apparatus has an image resolution (that is, voxel size) corresponding to the modality. The matrix size of 3D medical image data changes depending on, for example, the type of study. For example, volume data has a plurality of 16-bit voxels arrayed in, for example, 512 x 512 x 320 on Cartesian coordinates defined by an x-axis, a y-axis, and a z-axis. The plurality of voxels are arrayed along the respective axes at a predetermined interval of 0.5 mm. This corresponds to a spatial region of about 25 cm x 25 cm x 16 cm on the real space. This size is enough to include abdominal internal organs of interest such as the kidneys, liver, intestines, spleen, and pancreas. The medical image processing apparatus such as an image diagnostic apparatus converts the volume data into slice images such as transverse, coronal, or sagittal slice images. Note that a transverse slice section corresponds to the x-y plane, a coronal slice section corresponds to x-z plane, and a sagittal slice section corresponds to the y-z plane.

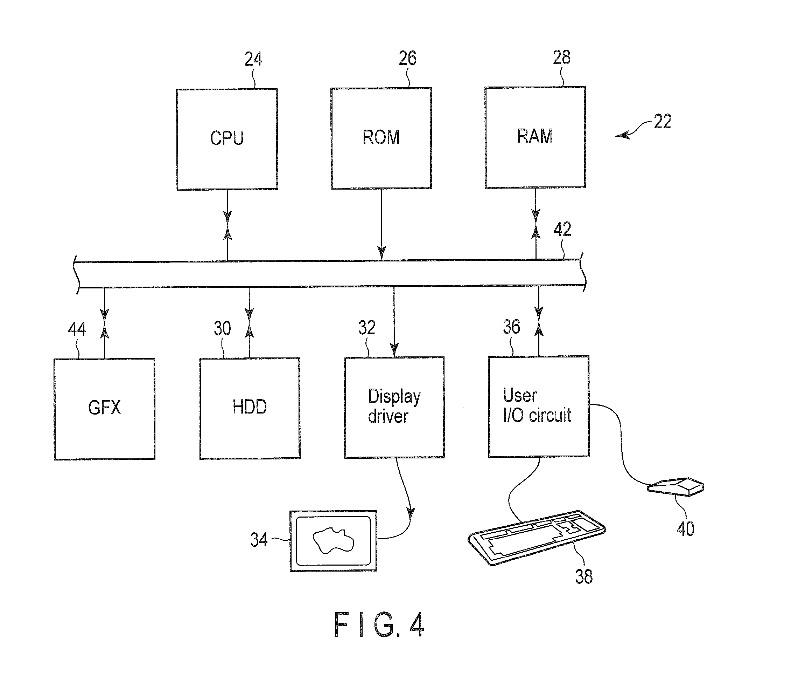

FIG. 4 is a block diagram schematically showing the arrangement of a medical image processing apparatus 22 according to this embodiment. The medical image processing apparatus 22 includes a CPU (Central Processing Unit) 24, a ROM (Read Only Memory) 26, a RAM (Random Access Memory) 28, a hard disk drive 30, a display driver 32, a display 34, and a user input/output circuit (user I/O) 36. The user I/O 36 is connected to a keyboard 38 and a mouse 40. These devices are connected via a common bus 42. The computer apparatus 22 also includes a GFX (Graphics Card) 44 connected via the common bus 42. The GFX 44 includes a GPU (Graphic Processing Unit) and a GPU memory (random access memory) coupled with the GPU, although not illustrated in FIG. 4.

The CPU 24 executes a program command stored in the ROM 26, the RAM 28, or the hard disk drive 30 and processes a medical image data set. The medical image data set is stored in the RAM 28 or the hard disk drive 30. The RAM 28 and the hard disk drive 30 will be referred to as a system memory altogether. The GPU executes a program command, receives a medical image data set from the CPU, and performs medical image processing for the received medical image data set.

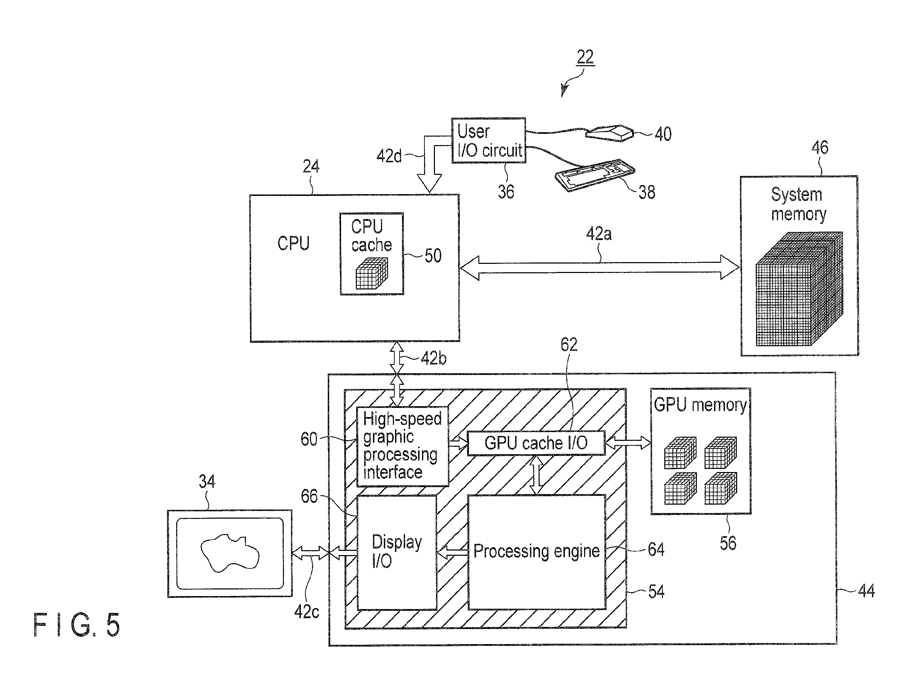

FIG. 5 is a view showing the detailed arrangement of the medical image processing apparatus shown in FIG. 4. Referring to FIG. 5, the RAM 28 and the hard disk drive 30 in FIG. 4 are illustrated as a system memory 46 altogether. The medical image data set is transferred from an image diagnostic apparatus and stored in the system memory 46. Referring to FIG. 5, the common bus 42 is illustrated as a plurality of separate common buses 42a to 42d to help indicating the data transfer route among the devices in the medical image processing apparatus 22. The first common bus 42a connects the system memory 46 and the CPU 24. The second common bus 42b connects the CPU 24 and the GFX 44. The third common bus 42c connects the GFX 44 and the display 34. The fourth common bus 42d connects the user I/O 36 and the CPU 24. The CPU 24 includes a CPU cache 50. The GFX 44 includes a GPU 54 and a GPU memory 56. The GPU 54 includes a high-speed graphic processing interface 60, a GPU cache input/output controller (GPU cache I/O) 62, a processing engine 64, and a display input/output controller (display I/O) 66. The processing engine 64 functions as an image processing unit that executes medical image processing according to this embodiment. The processing engine 64, for example, generates a display image based on medical image data.

In this embodiment, a medical image processing program installed in the medical image processing apparatus 22 is used to control the operations of the CPU 24 and the GPU 54. The medical image processing program is stored in a data storage medium such as a solid-state storage device or storage medium (a magnetic storage medium, an optical storage medium, or a magnetooptical storage medium by a disk or a tape). The medical image processing program may be supplied to the CPU 24 and the GPU 54 by a transfer medium (for example, a medium having a data storage medium, such as a telephone communication channel, a radio commendation channel, or an optical communication channel).

Medical image processing by the medical image processing apparatus 22 will be described below in detail.

Medical image processing according to this embodiment is associated with preset display. A preset defines the correspondence relationship between an observation target and display conditions. Examples of the observation target are anatomical parts, tissue characters, and medical supplies. The anatomical parts mean the vital tissues of a system of organs, individual organs, parts, or the like. Examples of the anatomical parts are the abdomen, lungs, abdominal blood vessels, fat, mediastinum, bones, liver, pectoral blood vessels, and head. The tissue characters mean the characters of human anatomy. Examples of the tissue characters are cancers, calcifications, and fibrosis. Examples of the medical supplies are the catheters, guide wires, balloons, stents, contrast agent, and drugs. Disease-associated substances are substances formed in a human body by diseases. The display conditions include a combination of a window level and a window width. The window level is the center value of the pixel value range of the observation target. The window width is defined by the pixel value range of the observation target. The display conditions are set to optimum values for each observation target in advance. The plurality of presets are stored in the system memory 46, the GPU memory 56, or the like.

The plurality of presets are displayed in a list format on the display 34. The user can, for example, select a desired preset from the displayed preset list by operating an input device such as the keyboard 38 or the mouse 40. The user can select the desired preset by moving a movable screen icon using the mouse, a track pad, or the like and performing point, click, touch screen, or another operation.

The selected preset is displayed on the display 34. For example, the selected preset is displayed as a histogram in which the display conditions such as the window level and the window width are displayed. The histogram is the pixel value frequency distribution of medical image data. The user can manually adjust the display conditions via the input devices 38 and 40. The adjusted display condition values are set to a new preset. For example, along with the progress of anatomical part observation, the user may adjust the display conditions to display the boundary between local anatomical structures at a high contrast. Since the human body has an enormous number of types of anatomical tissues, various kinds of contrast agent are introduced to obtain a high contrast in the image. As a result, the number of presets increases. In addition, since the vendor and the user add many specialty presets, the number of presets becomes extremely large.

For example, presets to be used for CT images are prepared for the following observation parts: (1) abdomen, (2) calcium, (3) lungs, (4) abdominal blood vessels, (5) fat, (6) mediastinum, (7) bones (hard), (8) liver, (9) pectoral blood vessels, (10) bones (soft), (11) liver (contrast imaging), and (12) head.

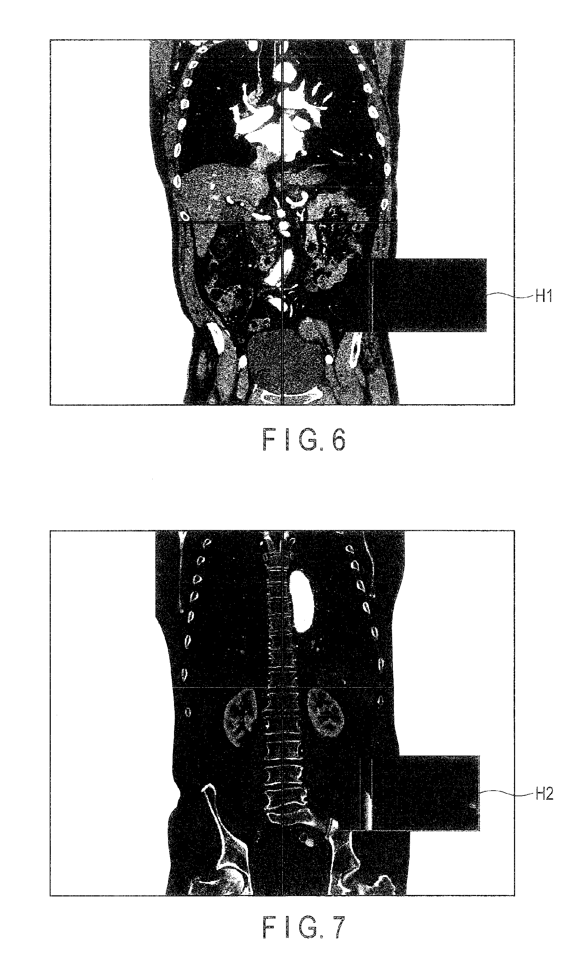

FIGS. 6, 7, and 8 are views showing display images generated by applying different presets to single medical image data. Note that the medical image data shown in FIGS. 6, 7, and 8 are CT volume data generated by the X-ray computed tomography apparatus, and the display images are slab MPR images based on the CT image data. Referring to FIGS. 6, 7, and 8, histograms H1, H2, and H3 based on the medical image data are displayed on the respective display images. The histograms H1, H2, and H3 show the display conditions. Note that the display images are generated by the processing engine 64 based on the medical image data.

FIG. 6 shows a slab MPR image based on CT volume data to which a preset for the abdomen is applied. The histogram H1 showing the window level and the window width appropriate for the abdomen is displayed on the slab MPR image shown in FIG. 6. As is apparent from FIG. 6, this slab MPR image allows to discriminate a plurality of anatomical structures concerning the abdomen. The plurality of anatomical structures concerning the abdomen have relatively close HU values. For this reason, the window width and the window level appropriate for the abdomen converge to a considerably narrow range. For example, the window level appropriate for the abdomen is set to 50 HU, and the window width appropriate for the abdomen is set to 400 HU. In this case, pixels belonging to the HU value range of 400 HU with a center value of 50 HU are displayed in shading of 256 gray levels. Pixels having HU values smaller than -150 HU are displayed in black, and pixels having HU values larger than 250 HU are displayed in white.

FIG. 7 shows a slab MPR image based on the same CT volume data as in FIG. 6 to which a preset for the bones is applied. In FIG. 7, the histogram H2 showing the window level and the window width appropriate for the bones is displayed. The bones are known to have high HU values. Hence, the window width and the window level are set to relatively high values. For example, the window level appropriate for the bones is set to 350 HU, and the window width appropriate for the bones is set to 1,200 HU. In this case, the denser the bones are, the whiter they are displayed in the slab MPR image. The anatomical features around the bones are identifiable but are displayed uniformly in light gray. Since the density value range to which non-bone structures belong is narrow, the difference between the non-bone structures cannot be identified.

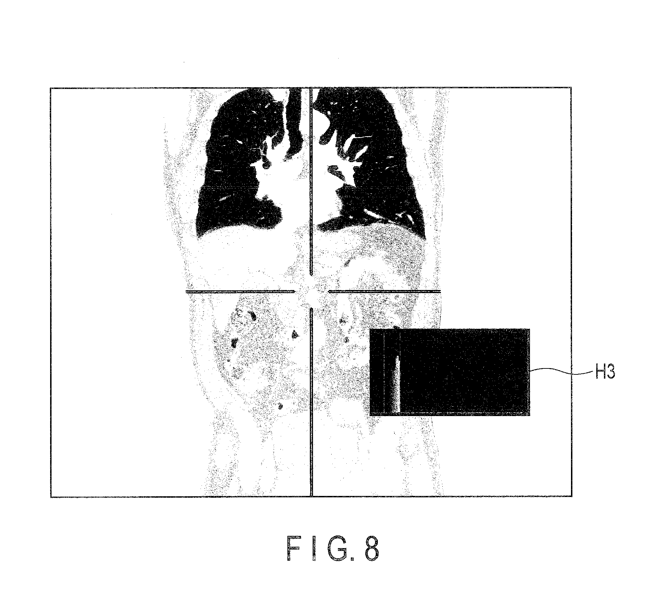

FIG. 8 shows a slab MPR image based on the same CT volume data as in FIGS. 6 and 7 to which a preset for the lungs is applied. In FIG. 8, the histogram H3 showing the window level and the window width appropriate for the lungs is displayed. Since the lungs contain a large quantity of air, it is important to show the contrast between the air in the lungs and the fine internal vessels on the slab MPR image. As can be seen from FIG. 8, the lungs and the vessels associated with them can easily be discriminated. Other various anatomical parts are uniformly displayed in white, and it is difficult to discriminate them.

The medical image processing apparatus 22 according to this embodiment does to display, on the display 34, all presets stored in the system memory 46 or the GPU memory 56. The medical image processing apparatus 22 selects, from all the presets, at least one preset suitable for the portion of interest designated by the user via the input device 38 or 40, and displays only the at least one selected preset. For example, when the user clicks the mouse button on the portion of interest in the initial display image, the medical image processing apparatus 22 identifies observation part candidates corresponding to the portion of interest based on the pixel values of the clicked portion of interest, and displays presets corresponding to the identified observation part candidates. The displayed presets are presented to user selection. That is, the presets presented to the user selection are the candidates of presets suitable for the portion of interest designated by the user. Hence, the presets presented to the user selection will be referred to as candidate presets hereinafter. The user designates an optimum preset from the displayed candidate presets via the input device 38 or 40. The medical image processing apparatus 22 generates a display image based on the medical image data in accordance with the window level and the window width of the designated preset. The generated display image is displayed on the display 34. Candidate preset selection processing executed by the medical image processing apparatus 22 as described above can be said to be a position-based method. The number of presets presented to the user selection is decreased by the automatic selection display.

The system memory 46 and the GPU memory 56 associate various kind of particular information with the presets to narrow down the candidate presets using the additional data and the image analysis result associated with the medical image data. Table 1 below is an example of the table of presets used in CT. Each preset in Table 1 is associated with the modality (for example, CT, MR, PET, or the like), anatomy (whole body, head, mediastinum, abdomen, leg, arm, or the like), the presence/absence of use of a contrast agent, and whether it is a default preset.

**Table 1**

| **Preset Name** | **Modality** | **Suitable Anatomy** | **Contrast Agent** | **Default Preset** |
|---|---|---|---|---|
| Abdomen | CT | Abdomen | No | No |
| Abdominal Blood Vessel | CT | Abdomen | No | No |
| Blood Vessel | CT | Whole body | Yes | No |
| Bone (Hard) | CT | Whole body | No | No |
| Bone (Soft) | CT | Whole body | No | Default |
| Calcium | CT | Whole body | No | No |
| Fat | CT | Whole body | No | No |
| Liver | CT | Abdomen | No | No |
| Liver (Contrast) | CT | Abdomen | Yes | No |
| Lung | CT | Abdomen | No | No |
| Mediastinum | CT | Abdomen | No | No |
| Thoracic Angio | CT | Abdomen | Yes | No |
| Head | CT | Head | No | No |

The data in Table 1 can be stored in the system memory 46 and the GPU memory 56 as either a lookup table or a database.

Table 1 includes three presets (blood vessels, pectoral blood vessels, and liver (blood vessels)) suitable for medical image data acquired while a contrast agent is being injected into the patient. In CT scan, the contrast agent is displayed like a high-density anatomical part. This is convenient for visualizing a small anatomical structure such as a blood vessel. The additional data associated with the medical image data includes information (presence/absence) about the presence/absence of use of the contrast agent. When data acquisition was performed during contrast agent injection, the additional data includes information (present) representing that the contrast agent was used. Such additional data is stored in the header portion of the DICOM file, as described above. The additional data concerning the presence/absence of use of a contrast agent is used to include or exclude presets suitable for the contrast agent in or from the candidate presets. The GUI may provide the user an environment for specifying whether a contrast agent was used.

As shown in Table 1, the additional data associated with the medical image data may include the anatomical part. This allows the processing engine 64 to automatically exclude a preset unsuitable for the anatomical part stored in the header portion of the DICOM file. For example, when displaying medical image data concerning the abdomen, presets associated with the anatomical parts such as the head other than the abdomen are excluded. The GUI may display, for the user, an environment for designating an anatomical part. In this case, presets are excluded based on manual input (for example, input of information representing head study) by the user.

As shown in Table 1, one default preset is set in each modality. As will be described later, when initially displaying medical image data, the display image based on the medical image data is displayed in accordance with the display conditions corresponding to the default preset.

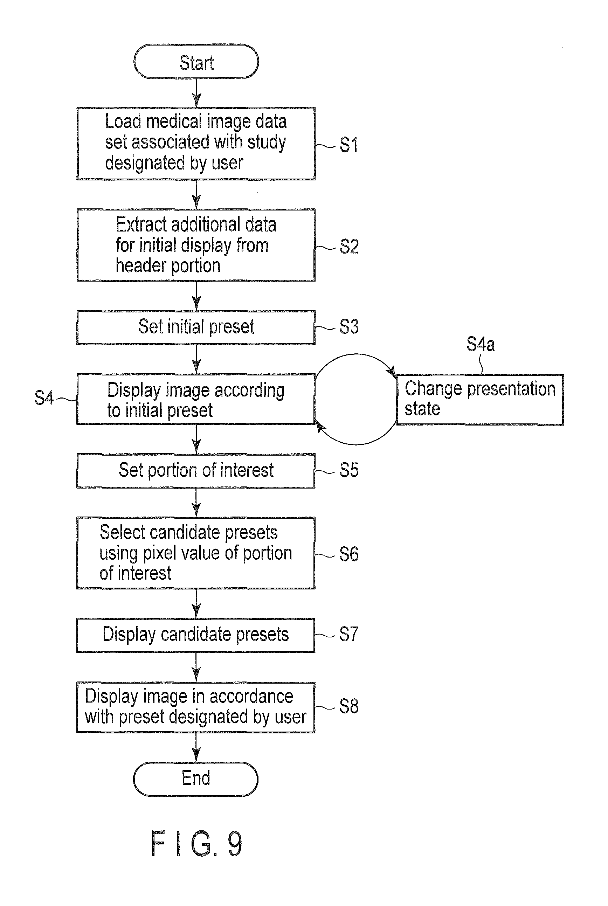

Candidate preset display processing executed by the processing engine 64 will be described next in detail with reference to FIG. 9. FIG. 9 is a flowchart showing the typical procedure of candidate preset display processing executed by the processing engine 64.

In response to an instruction to start the medical image processing according to this embodiment, which is input by the user via the input device 38 or 40, the processing engine 64 activates the medical image processing program. The user selects, for example, an study of an interpretation target via the input device 38 or 40. The selected study will be referred to as interpretation study hereinafter.

In step S1, the processing engine 64 loads, from the GPU memory 56, a medical image data set for the interpretation study selected by the user via the input device 38 or 40. The medical image data set for the single study can be read out from either a single DICOM file or a plurality of DICOM files.

In step S2, the processing engine 64 searches for additional data stored in the header portion of the medical image data set read out in step S1 using a search keyword to select an initial preset. The search keyword can be set to an arbitrary particular such as the modality, the presence/absence of use of a contrast agent, or the anatomical part. The additional data for initial display, which is associated with the search keyword, is extracted. For example, when displaying the medical image data by the default preset of the modality used for the interpretation study, modality data is extracted from the additional data stored in the header portion. In CT study, the modality "CT" is extracted. In this case, only presets for the CT are read out from the GPU memory 56, and presets for the modalities such as MR other than CT are not read out. The search keyword can be preset or set by the user via the input device 38 or 40.

In step S3, the processing engine 64 specifies the initial preset of the presets stored in the GPU memory 56 in accordance with the additional data extracted in step S2 for initial display. More specifically, the processing engine 64 specifies the preset associated with the additional data for initial display from the plurality of presets stored in the GPU memory 56. The processing engine 64 sets the specified preset as the initial preset. However, if a plurality of presets are specified, the default preset of the presets is set as the initial preset. For example, if the additional data for initial display is "CT", the default preset of the presets associated with "CT" is set as the initial preset. When a preset having specific display conditions is stored in the header portion as, for example, part of operation state data stored from the previous session, this preset is selected. The selected preset is registered in the GPU memory 56. If the name of the preset is stored in the header portion, the preset associated with the name is set as the initial preset. The initial preset may be a preset set by the user in advance. A preset that wholly covers the actual or potential data range, for example, a data range having a linear sensitivity throughout may be selected as the initial preset. The initial preset may be designated by the user via the input device 38 or 40 from all presets associated with the additional data for initial display in step S3.

In step S4, the processing engine 64 generates a display image based on the medical image data in accordance with the display conditions corresponding to the initial preset. The processing engine 64 causes the display 34 to display the display image by controlling the display I/O. When the display image is displayed, the processing engine 64 receives an arbitrary change for the display image presentation state from the user. For example, the user executes an input device operation for pan, tilt, zoom, and sculpting (image manipulation) via the input device 38 or 40. The processing engine 64 executes the image manipulation such as pan, tilt, zoom, and sculpting in accordance with the input device operation and changes the display image presentation state. The subset of patient data set is thus changed in the display image on the display 34. For example, in volume data of the whole body, the display range of the display image based on the volume data is limited to the head. Specific organ selection by the user via the input device 38 or 40 can also be included in the image manipulation.

The screen of the display 34 may be changed from the last refresh as the result of presentation state change by the user in step S4a. For example, when the user has performed image manipulation for the tissue type, the processing engine 64 evaluates that the preset specialized to the tissue type before the image manipulation is not appropriate. When the user has manipulated the display image presentation state and selected a specific organ, the processing engine 64 evaluates that a preset specialized to the selected organ is appropriate.

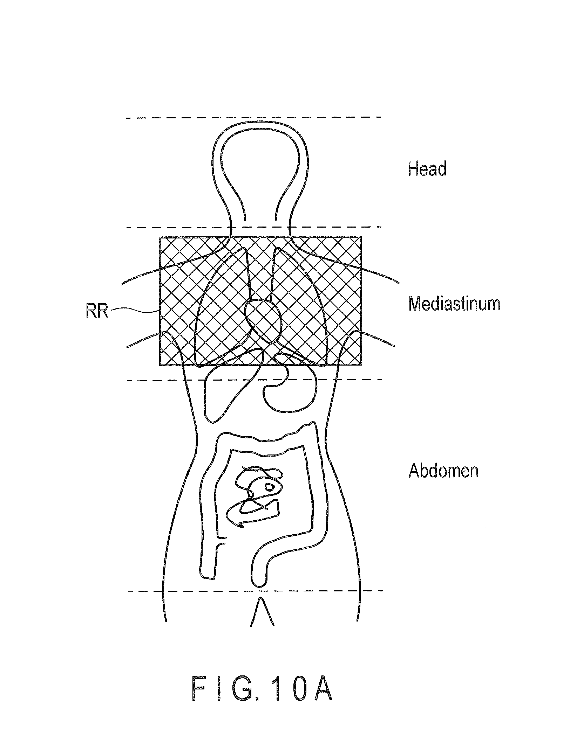

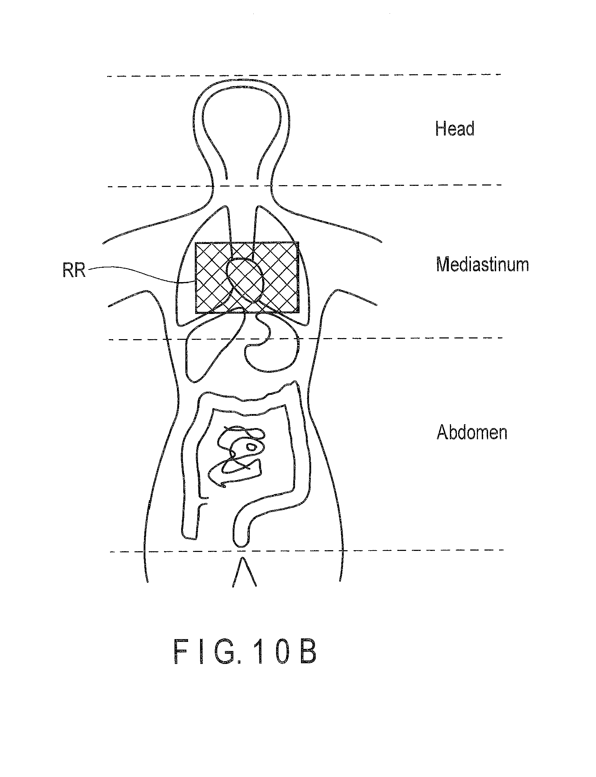

FIGS. 10A and 10B are views showing an example of image manipulation. FIG. 10A is a schematic view of a human body including several major organs. A rectangular region RR indicates the display range designated by the user. Referring to FIG. 10A, the display range RR indicates the whole mediastinum including the lung region and the heart region. The user can change the presentation state by zooming the display range RR on the heart region, as schematically shown in FIG. 10B. FIG. 10B is the same as FIG. 10A except that the display range RR is zoomed in. The user can repetitively change the presentation state of the display image on the display 34 as represented by the loop of steps S4 and S4a in FIG. 9. The image manipulation need not always be only the simple zoom and may include, for example, pan. The lungs may be deleted, or the heart may be selected as the target of interest by the image manipulation.

In step S5, the processing engine 64 waits for designation of the portion of interest by the user via the input device 38 or 40. The user designates an arbitrary portion of interest on the display image via the input device 38 or 40. The input portion of interest represents the region in which the user is interested. The processing engine 64 sets the part in the medical image data corresponding to the portion of interest on the display image as the portion of interest. Note that the portion of interest can be either a point designated by the user or a region of a predetermined size including the point designated by the user.

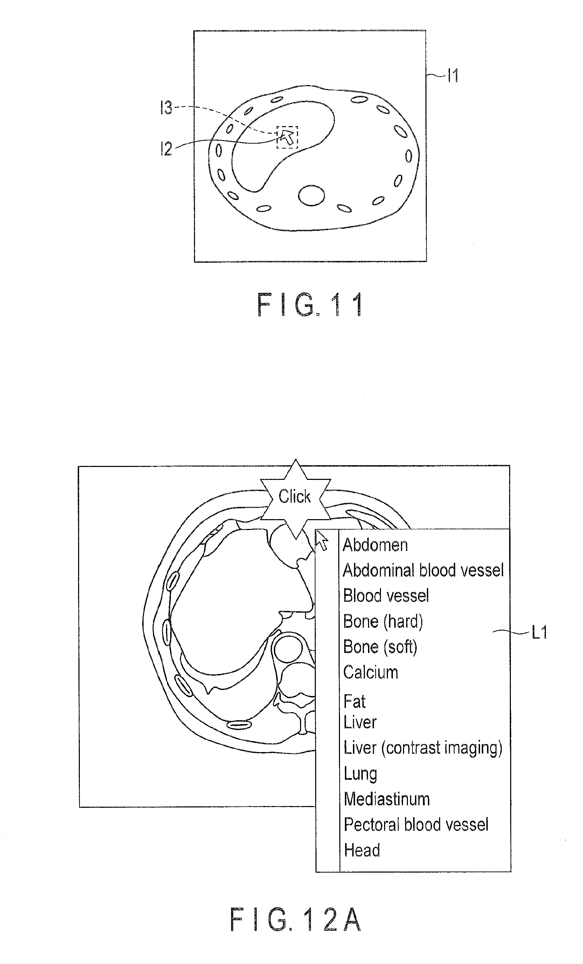

FIG. 11 is a view for explaining designation of the portion of interest. As shown in FIG. 11, a display image I1 is displayed on the display 34 in step S5. Referring to FIG. 11, the display image I1 concerns the abdomen of the patient. The user designates the portion of interest by operating a cursor 12 via the input device 38 or 40. The display 34 displays a frame 13 indicating the designated portion of interest. The user can thus confirm the range of the portion of interest. Note that the shape of the portion of interest is not limited to the rectangular shape but may have an arbitrary shape. The user can arbitrarily set the shape and size of the portion of interest via the input device 38 or 40.

The portion of interest may be displayed in colors. For example, a deep yellow color value is assigned to pixels corresponding to the blood vessel system. A transparent red color value is assigned to pixels corresponding to the soft tissues. A light yellow color value is assigned to pixels corresponding to the bones. The display 34 displays the portion of interest in colors according to the assigned colors. The portion of interest and the remaining regions on the display image are displayed in different colors.

In step S6, the processing engine 64 selects at least one candidate preset corresponding to at least one observation part according to the portion of interest from the plurality of presets stored in the GPU memory 56 using the pixel values of the pixels included in the set portion of interest. The processing in step S6 will be described below in detail. Note that the portion of interest is assumed to be a region formed from a plurality of pixels. First, the processing engine 64 determines the candidates of the observation part corresponding to the portion of interest using the pixel values of the plurality of pixels included in the portion of interest of the medical image data. The GPU memory 56 stores a table that associates a standard pixel value range to which an observation part belongs with each of a plurality of observation parts. This table will be referred to as an observation part-pixel value range table hereinafter. Table 2 below is an example of the observation part-pixel value range table for CT.

**Table 2**

| **Air (<-100)** | **Soft Tissues (-100 to 100)** | **High-Density Tissue (>100)** |
|---|---|---|
| Lung | Abdomen | Blood Vessel |
| Mediastinum | Abdominal Blood Vessel | Bone (Hard) |
| Pectral Blood Vessel | Liver | Bone (Soft) |
| | Liver (Contrast) | Calcium |
| | Mediastinum | |
| | Head | |
| | Fat | |

In Table 2, the observation parts are roughly classified into three types by the pixel value (HU value) range. More specifically, the HU value range smaller than -100 is classified as air. The HU value range from -100 to +100 is classified as soft tissues. The HU value range larger than +100 is classified as high-density tissues. Examples of the observation parts belonging to air are the lungs, mediastinum, and pectoral blood vessels. Examples of the observation parts belonging to soft tissues are the abdomen, pectoral blood vessels, liver, liver (contrast imaging), mediastinum, head, and fat. Examples of the observation parts belonging to high-density tissues are the blood vessels, bones (hard), bones (soft), and calcium.

The processing engine 64 determines the pixel value of the portion of interest based on the pixel values of the plurality of pixels included in the portion of interest. For example, the processing engine 64 sets the statistics of the pixel values of the plurality of pixels included in the portion of interest as the pixel value of the portion of interest. The statistic is set to the average value, the intermediate value, the minimum value, or the maximum value of the pixel values of the plurality of pixels included in the portion of interest. The processing engine 64 searches the observation part-pixel value range table using the statistics of the portion of interest as the search keyword, and specifies the observation part associated with the search keyword. Note that if the observation parts cannot be narrowed down to a few only by the statistics, the processing engine 64 determines the observation parts corresponding to the portion of interest using the position of the portion of interest in the medical image data as well as the statistics of the portion of interest. As the position of the portion of interest, for example, a combination of the imaging part associated with the medical image data and the position coordinates of the portion of interest in the medical image data can be used. An anatomical part corresponding to a combination of the imaging part and the position coordinates in standard image data is empirically known. The correspondence relationships can be accumulated in the GPU memory 56 as a table or an anatomical atlas. The processing engine 64 determines the observation part candidates from the pixel value and position of the portion of interest using the correspondence relationship and the observation part-pixel value range table. Note that the data of the imaging part can be input by the user via the input device 38 or 40 or extracted from the additional data associated with the medical image data.

The processing engine 64 may identify the observation part corresponding to the portion of interest using not the statistic but the histogram of the portion of interest. The histogram is the pixel value frequency distribution. The GPU memory 56 stores a histogram library that associates a standard histogram with each of a plurality of observation parts. The processing engine 64 calculates the histogram based on the plurality of pixel values included in the portion of interest and collates the histogram of the portion of interest with the plurality of standard histograms in the histogram library. The processing engine 64 specifies a standard histogram similar to the histogram of the portion of interest and identifies the observation part associated with the specified standard histogram. The identified observation part is set as the candidate observation part corresponding to the portion of interest.

When the candidate observation part is determined, the processing engine 64 selects a preset corresponding to the determined candidate observation part from the plurality of presets stored in the GPU memory 56. The selected preset is set as the candidate preset. In this way, the processing engine 64 selects the candidate preset using the pixel value of the portion of interest. Either one or a plurality of candidate presets can be set. To do the description below in detail, assume that a plurality of candidate presets are selected.

In step S7, the processing engine 64 displays the plurality of candidate presets in a list format on the display 34. The plurality of candidate presets are displayed while being superimposed on the display image. The plurality of candidate presets are displayed, for example, near the portion of interest set in step S5. The list display of the plurality of candidate presets will be described later.

In step S8, the processing engine 64 waits for designation of a single preset of the candidate presets by the user via the input device 38 or 40. The user designates, via the input device 38 or 40, a preset optimum for the portion of interest of the candidate presets. The processing engine 64 generates the display image based on the medical image data in accordance with the designated optimum preset. More specifically, the processing engine 64 first performs image processing of the medical image data designated by the user to generate 2D medical image data. Example of the image processing are volume rendering, surface rendering, pixel value projection processing, and MPR (Multi-Planar Reconstruction). The processing engine 64 applies the display conditions, that is, the window level and the window width belonging to the designated optimum preset to the 2D medical image data to perform density conversion of the pixel value of each pixel of the medical image data and generate a display image according to the window level and the window width. The processing engine 64 displays the generated display image on the display 34.

The candidate preset display processing executed by the processing engine 64 thus ends.

As described above, the processing engine 64 can display only the candidate presets suitable for the portion of interest in the medical image data using the pixel value of the portion of interest. The user selects an optimum preset from the small number of candidate presets. Hence, in this embodiment, the user's labor of selecting the preset can be reduced as compared to the related art in which a preset is selected from all presets.

Note that to further reduce the user's labor of selecting the preset, the plurality of candidate presets are preferably displayed in an order corresponding to the reliability. The reliability is calculated by the processing engine 64 for each of the plurality of candidate presets. The reliability is defined as the likelihood that the portion of interest designated by the user is a candidate observation part corresponding to each preset. The reliability of a candidate preset is defined by, for example, the shift between the statistics of the portion of interest and the standard pixel value or the shift between the occupation range of the candidate observation part in the medical image data and the standard occupation range. A case in which the reliability is defined by the shift between the statistics of the portion of interest and the standard pixel value will be described first. The standard pixel value is an empirically determined standard pixel value that the observation part can have. For example, a standard pixel value is associated with each of the plurality of observation parts in the observation part-pixel value range table. In the observation part determination processing, the processing engine 64 calculates the difference between the statistics of the portion of interest and the standard pixel value associated with the determined candidate observation part in the observation part-pixel value range table. This difference value is set as the reliability. The smaller the difference value is, the higher the reliability is. A case in which the reliability is defined by the shift between the occupation range of the candidate observation part in the medical image data and the standard occupation range will be described next. The occupation range of the candidate observation part in the medical image data is, for example, the range occupied by the candidate observation part in the medical image data. The standard occupation range is the range occupied by a standard observation part in standard medical image data. The standard occupation range corresponds to the occupation range of each of the plurality of observation parts in the above-described atlas. In the observation part determination processing, the processing engine 64 calculates the area of the difference between the occupation range of the determined candidate observation part and the occupation range of the candidate observation part in the atlas. The area value is set as the reliability. The smaller the area value is, the higher the reliability is. In step S6, the processing engine 64 calculates the reliability for each candidate observation part. Note that the type of reliability can arbitrarily be set by the user via the input device 38 or 40. The display 34 displays the plurality of candidate presets arranged in the order of the reliability of the candidate observation part. For example, as the reliability of the candidate observation part rises, the candidate preset is displayed in the higher rank of the list.

Sorting of the candidate presets is not limited to sorting by reliability. For example, the candidate presets may be sorted by the preset use count. The use count is calculated by the processing engine 64 based on a log accumulated for a predetermined unit such as each user, each predetermined period, or each study. Each of the plurality of presets is stored in the GPU memory 56 or the like in association with the use count.

In the above description, a plurality of presets are selected in step S6. However, the embodiment is not limited to this. For example, the processing engine 64 may select a single candidate preset in step S6. In step S7, the display 34 displays the single selected candidate preset. Alternatively, in step S7, the display 34 may immediately display the display image generated by the processing engine 64 based on the medical image data in accordance with the single selected candidate preset.

After the display of the display image in step S8, the user may designate another portion of interest on the display image via the input device 38 or 40. If the portion of interest is designated again, the processing engine 64 returns to step S6 of FIG. 9. The processing engine 64 then selects candidate presets suitable for the newly designated portion of interest, and displays the selected candidate presets in the list format.

After the display of the display image in step S8, step S4a may be performed. That is, the user may change the presentation state of the display image in step S8 via the input device 38 or 40.

The candidate presets are updated in response to the change in the presentation state in step S4a or the setting of the part of interest in step S5. For example, when the display image is panned or zoomed in, an anatomical structure appears in the display region or disappears from the display region. Refresh is performed in response to designation of the portion of interest by the user via the input device 38 or 40. With this mechanism, the refresh is efficiently done. The refresh can be done in response to a user's manual instruction input by, for example, pressing a separate button or the like on the GUI displayed on the display 34.

The optimum preset selected by the user in step S8 is saved in the GPU memory 56 in association with the medical image data. More specifically, the optimum preset is saved in the header portion of the medical image data set. This allows to use the optimum preset initially or by default when the medical image data set is read out in the future. The default preset may be used at the time of reread of the medical image data set. More specifically, the default preset may be used when a single user executes the reread, when any other user executes the reread independently of the user, or a specific user (a user who has not already stored the default preset specialized to the user in the header portion of the medical image data set) executes the reread.

Any other scheme that allows the user to identify a pixel may be used. For example, instead of identifying the individual pixels of one slice image by "clicking" on them, the user may specify a pixel range by "clicking" twice on diagonal vertices of a rectangle or the center and a point on the circumference of a circle or any other method and identify the pixels. The range is identified on the touch screen by, for example, a gesture using the thumb and the index finger. All pixels around the range (that is, the voxel in the 3D medical image data corresponding to the pixels of the display image) are evaluated as identified.

Display examples of the candidate presets will be explained below.

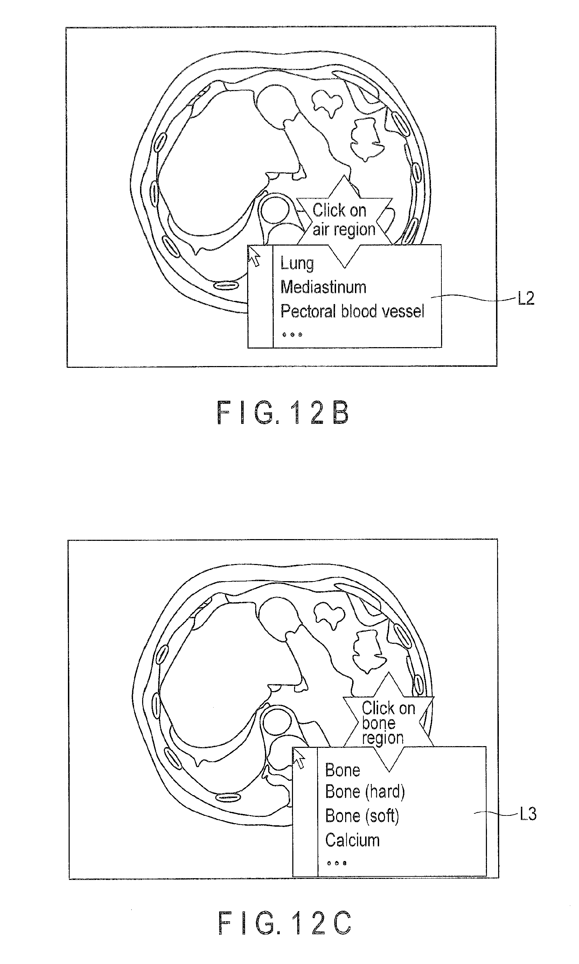

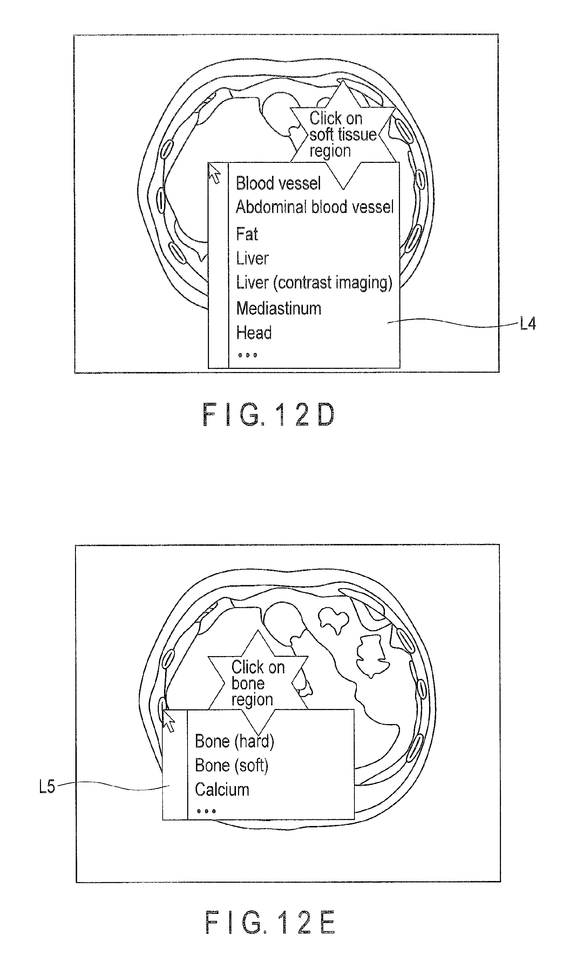

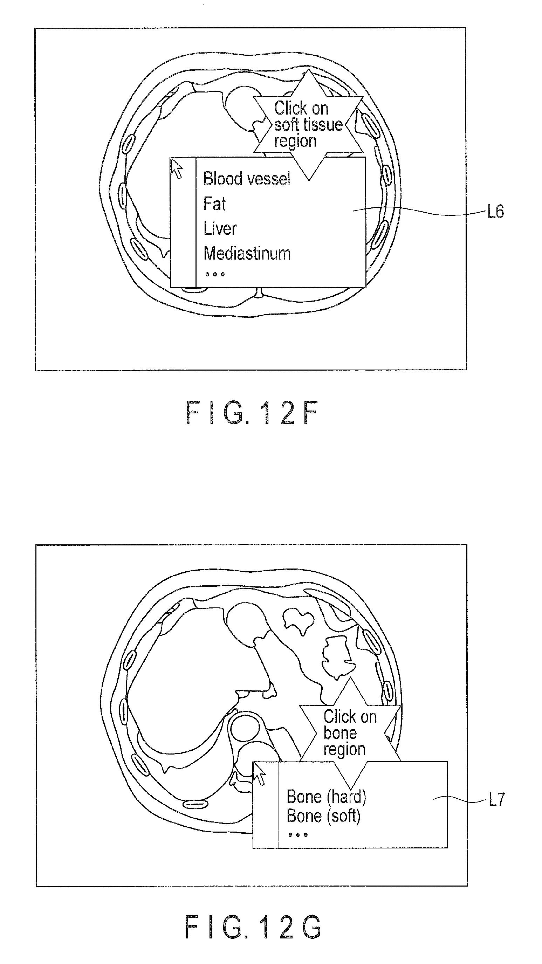

FIGS. 12A, 12B, 12C, 12D, 12E, 12F, and 12G are views showing display examples of the candidate presets. All the display images included in FIGS. 12A, 12B, 12C, 12D, 12E, 12F, and 12G are derived from single medical image data. All the display images included in FIGS. 12A, 12B, 12C, 12D, 12E, 12F, and 12G are the axial slice images of the patient's abdomen. Each axial slice image is a display image based on raw data acquired by the conventional method in CT scan. The candidate preset list is displayed when, for example, the user "clicks" on the portion of interest.

FIG. 12A shows a conventional list L1 in which all presets are displayed. Presenting the list L1 including all presets to the user in this way is not ideal. This is because seeing many presets is time-wasting for the user. Note that the list L1 does not include a custom menu arbitrarily set by the user not to be long.

FIG. 12B is a view showing a candidate preset list L2 displayed when the user clicks on an air region in the display image. In this case, candidate presets to display air, that is, an HU value range smaller than -100 HU by gray scale are selected, and the list L2 of the selected candidate presets is displayed on the display 34.

FIG. 12C is a view showing a candidate preset list L3 displayed when the user clicks on a bone region in the display image. In this case, candidate presets to display the bones, that is, an HU value range larger than +100 HU by gray scale are selected, and the list L3 of the selected candidate presets is displayed on the display 34.

FIG. 12D is a view showing a candidate preset list L4 displayed when the user clicks on a soft tissue region in the display image. In this case, candidate presets to display the soft tissues, that is, an HU value range from -100 HU (inclusive) to +100 HU (inclusive) by gray scale are selected, and the list L4 of the selected candidate presets is displayed on the display 34.

Lists of highly selected candidate presets will be described next with reference to FIGS. 12E, 12F, and 12G.

FIG. 12E is a view showing an advanced candidate preset list L5 displayed when the user clicks on a bone region in the display image. Presets that do not belong to the HU range of a high-density structure selected by the user are also deleted from the list L5 shown in FIG. 12E. In addition, the preset for the contrast agent that is classified as a high-density structure is deleted from the list L5 shown in FIG. 12E. The preset for the contrast agent is excluded from the candidate presets using, for example, a user input or additional data representing that the medical image data was acquired without using the contrast agent. Alternatively, the preset for the contrast agent may be excluded from the candidate presets using the position of the bone region, as described above.

FIG. 12F is a view showing an advanced candidate preset list L6 displayed when the user clicks on a soft tissue region in the display image. The preset for the liver (contrast imaging) is excluded from the list L6. The preset for the liver (contrast imaging) and the preset for the abdominal blood vessels are excluded using, for example, a user input or additional data representing that the medical image data was acquired without using the contrast agent. The preset for the head is also excluded from the list L6 shown in FIG. 12F, as compared to the list L4 show in FIG. 12D. For example, the preset for the head is excluded using the positions of the soft tissues.

As described above, the candidate observation part is identified using the pixel value of the portion of interest. However, the candidate observation part may be identified using segmentation data for the portion of interest. The segmentation data is generated by performing segmentation processing for the medical image data. More specifically, the user designates the portion of interest on the display image via the input device 38 or 40. The processing engine 64 sets the portion of interest as a seed point and performs segmentation processing for the medical image data based on the set seed point. More specifically, the processing engine 64 searches for a pixel around the seed point and integrates pixels that meet an integration condition based on the pixel value of the seed point. The plurality of pixels thus integrated form a set of pixels similar to the pixel of the seed point, which is regarded as a pixel region associated with the anatomical part to which the portion of interest belongs. The processing engine 64 identifies the anatomical part of the pixel region based on the pixel value, position, and the like of the pixel region. More specifically, all constituent elements of the human body such as the bones, blood vessels, lungs, liver, heart, and brain can be applied as the anatomical part. The pixel region of the anatomical part will be referred to as an anatomical region hereinafter. A combination of the identifier of the anatomical region and that of the anatomical part constitutes a segmentation.

When the segmentation processing is used, the candidate observation target is determined based on the pixel value of the anatomical region including the portion of interest determined by the segmentation processing in step S6. The segmentation data has two application purposes. In the first application purpose, a pixel value such as HU used to select a preset is obtained from the anatomical region determined by the segmentation processing based on the seed point designated by the user. For example, when the user clicks on a point on the liver region as the portion of interest, the pixel value is obtained from the entire liver region. In the second application purpose, a preset is selected based on the anatomical similarity. For example, when the user clicks on a point on the heart region, the preset for the liver is excluded. The preset for the heart and the preset for the liver may be displayed in different colors or transparency levels. The segmentation data can be prepared in advance, as described above, or calculated by on-the-fly as part of preset selection processing in response to designation of the portion of interest (or a portion of non-interest). The on-the-fly segmentation is executed in accordance with a quantitative condition or the type of user input of the portion of interest. For example, the segmentation is preferably performed only when the upper limit (or lower limit) of the selection count is 4, 5, 6, 7, 8, 9, or 10. As another example, the on-the-fly segmentation may be performed only in case of negative selection. In negative selection, that is, selection of a portion of non-interest, the number of thresholds of the negative selection preferably equals the number of thresholds in positive selection, for example, 2, 3, 4, 5, 6, 7, 8, 9, or 10.

FIG. 12G is a view showing an advanced candidate preset list L7 displayed when the user clicks on a bone tissue region in the display image. The candidate presets included in the list L7 are narrowed down using segmentation data. When the user clicks on one point on the bone region, a local region including the one clicked point is set as the portion of interest. The processing engine 64 performs segmentation processing for the medical image data to segment a plurality of anatomical regions included in the medical image data. An anatomical region including the portion of interest is thus recognized as a bone region. The processing engine 64 selects the preset for the bones from the candidate presets and displays the selected candidate preset on the display 34.

Note that the display image according to this embodiment is not limited to the axial slice image. For example, the display image may be a coronal slice image or a sagittal slice image. The display image can be a volume rendering image, a surface rendering image, or a pixel value projection image. As the pixel value projection image, a maximum value projection image, a minimum value projection image, an average value projection image, or the like can be applied.

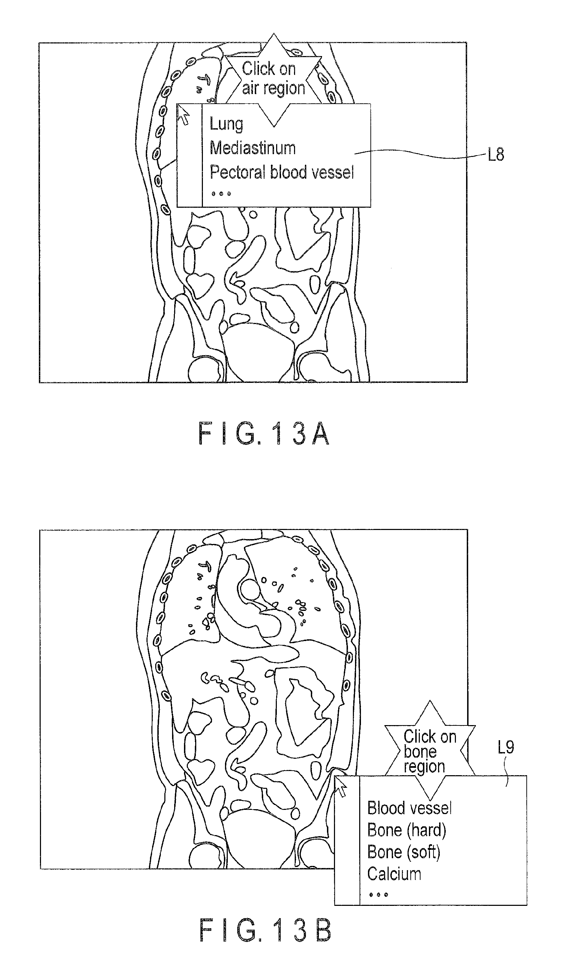

FIG. 13A is a view showing a display example of a candidate preset list L8 displayed when the user clicks on an air region in a coronal slice image. Note that the coronal slice image shown in FIG. 13A is generated based on the same medical image data concerning the abdomen as the medical image data shown in FIGS. 12A, 12B, 12C, 12D, 12E, 12F, and 12G. The list L8 includes candidate presets for air regions in the abdomen.

FIG. 13B is a view showing a display example of a candidate preset list L9 displayed when the user clicks on a bone region in the coronal slice image. Note that the coronal slice image shown in FIG. 13B is generated based on the same medical image data concerning the abdomen as the medical image data shown in FIGS. 12A, 12B, 12C, 12D, 12E, 12F, and 12G. The list L9 includes candidate presets for bone regions in the abdomen.

An MPR image is handled like the above-described 2D image. The user demonstrates, in a list of selected presets, that he/she is interested in air or bones. When a non-slab MPR image is used, the pixel value of the original medical image is directly returned to a point indicated by the user using, for example, a mouse. However, the MPR image is generated by sampling many voxels included in volume data. For example, when the original slice images are arrayed at an interval of 1 mm, the user can construct a 5-mm thick slab MPR image. In this case, five images are used to determine the pixel value of the slab MPR image. The mechanism for determining the pixel value of the slab MPR image is determined in accordance with the projection mode. In general, following three projection modes are used to generate a slab MPR image.
· AveIP (average value projection): the average voxel value is used.
· MIP (maximum value projection): the maximum voxel value is used.
· MinIP (minimum value projection): the minimum voxel value is used.

When sampling points from a slab MPR image, the collection value of the pixel values of the sampling points is used as the value calculated in the projection mode. The collection value is used to select candidate presets having display conditions suitable for visualizing an anatomical region.

This embodiment has been described using gray scale display as a detailed example. However, color scale display is also possible. In this case, a combination of a window level and a window width for color scale display is used as display conditions. The display conditions are not limited to the window level and the window width. For example, the transparency, the rate of change in the density value with respect to the pixel value, the rate of change in the transparency with respect to the pixel value, and segmentation information may be included in the display conditions.

The medical image processing method according to the embodiment and a medical image processing program associated with it are incorporated in, for example, a volume rendering application.

This specification discloses a medical image processing program that holds a mechanically readable instruction to execute the medical image processing method according to the embodiment.

This specification discloses a medical image processing apparatus that is operable to load and execute a mechanically readable instruction to execute the medical image processing method according to the embodiment.

This specification discloses a medical image processing program. The medical image processing program that holds a mechanically readable instruction to execute the medical image processing method according to the embodiment is stored in the hard disk drive 30, the ROM 26, and the RAM 28 shown in FIG. 4, the system memory 46 and the GPU memory 56 shown in FIG. 5, and the servers 13 and 18 shown in FIG. 1. The medical image processing program may be stored in a disk-like storage device such as a CD or DVD, or a USB flash memory.

The computer that is operable to load and execute a mechanically readable instruction to execute the medical image processing method according to the embodiment includes the computer shown in FIG. 4, the computer shown in FIG. 5, and the individual constituent elements. The individual constituent elements include, for example, a terminal or a combined apparatus of the computer network system shown in FIG. 1. An example of the combined apparatus is the file server 13 or 18 connected to at least one of terminals or computers incorporated in a medical imaging apparatus.

As the image diagnostic apparatus according to the embodiment, an X-ray computed tomography apparatus has been described as a main detailed example. However, the embodiment is not limited to this. The image diagnostic apparatus according to the embodiment is also applicable to an X-ray diagnostic apparatus, a magnetic resonance imaging apparatus, an ultrasonic diagnostic apparatus, a positron emission tomography apparatus (PET scanner), a single photon emission computed tomography apparatus (SPECT scanner), a PET/CT apparatus, and the like.

As the medical image data according to the embodiment, CT image data acquired by the X-ray computed tomography apparatus has been described as a main detailed example. However, the embodiment is not limited to this. The medical image data according to the embodiment is also applicable to medical image data acquired by an image diagnostic apparatus such as an X-ray diagnostic apparatus, a magnetic resonance imaging apparatus, an ultrasonic diagnostic apparatus, a positron emission tomography apparatus (PET scanner), a single photon emission computed tomography apparatus (SPECT scanner), or a PET/CT apparatus.

According to the embodiment, it is possible to provide a medical image processing apparatus which enable to reduce the user's labor for image display.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A medical image processing apparatus **characterized by** comprising:
a storage unit configured to store a plurality of presets, each of the presets defining a correspondence relationship between an observation target and a display condition;
a setting unit configured to set a portion of interest in medical image data in accordance with an instruction from a user;
a selection unit configured to select at least one candidate preset corresponding to at least one candidate observation target according to the portion of interest from the plurality of presets using a pixel value of a pixel included in the set portion of interest; and
a display unit configured to display the at least one selected candidate preset.

2. The apparatus according to claim 1, **characterized in that** the selection unit determines the at least one candidate observation target and selects the at least one candidate preset corresponding to the at least one determined candidate observation target from the plurality of presets using the pixel value of the pixel included in the set portion of interest.

3. The apparatus according to claim 1, **characterized in that** the selection unit selects the at least one candidate preset further using additional data associated with the medical image data.

4. The apparatus according to claim 3, **characterized in that** the additional data includes at least one of modality information, contrast agent information, anatomical information that defines a body portion of an imaging target, a diagnostic purpose, and a user's clinical status.

5. The apparatus according to claim 1, **characterized in that** the selection unit selects the at least one candidate preset further using segmentation data that provides anatomical information about the portion of interest.

6. The apparatus according to claim 1, **characterized in that** the display unit displays the at least one candidate preset while sorting the at least one candidate preset by reliability.

7. The apparatus according to claim 1, **characterized in that**
the at least one candidate preset includes the plurality of candidate presets, and
the plurality of presets are sorted by reliability and includes a preset optimum for the portion of interest.

8. The apparatus according to claim 1, **characterized in that** the display unit displays the at least one candidate preset near the portion of interest.

9. The apparatus according to claim 1, **characterized in that** the storage unit stores the at least one candidate preset in association with the medical image data.

10. The apparatus according to claim 1, **characterized in that** the display unit displays a display image based on the medical image data in accordance with the display condition for a preset designated by the user of the at least one candidate preset.

11. The apparatus according to claim 1, **characterized in that** when the at least one candidate preset is a single candidate preset, the display unit immediately displays a display image based on the medical image data in accordance with the display condition for the single candidate preset.

12. The apparatus according to claim 1, **characterized in that** the storage unit stores a preset designated by the user of the at least one candidate preset in association with the medical image data.

13. The apparatus according to claim 1, **characterized in that** the portion of interest includes a plurality of pixels close to a pixel designated by the user.

14. The apparatus according to claim 1, **characterized in that** the portion of interest includes a plurality of pixels determined by segmentation based on a seed point designated by the user.

15. The apparatus according to claim 1, **characterized in that** the selection unit selects the at least one candidate preset further using, as the pixel value of the pixel, of a plurality of pixels included in the portion of interest in the medical image data and a statistics based on the pixel values of the plurality of pixels.
